# EUROPEAN PATENT SPECIFICATION

(11) **EP 3 397 134 B1**
(45) Date of publication and mention of the grant of the patent: **22.04.2020**
(21) Application number: 16829035.1
(22) Date of filing: 28.12.2016
(51) Int. Cl.: A61B 1/303, A61B 1/32

(54) **ERGONOMICALLY DESIGNED VAGINAL SPECULUM**
ERGONOMISCH GESTALTETES VAGINALSPEKULUM
SPÉCULUM VAGINAL À CONCEPTION ERGONOMIQUE

(30) Priority: 29.12.2015 US 201562272616 P; 21.01.2016 US 201662281690 P; 18.03.2016 US 201662310602 P
(43) Date of publication of application: 07.11.2018
(73) Proprietor: Ceek Women's Health, Inc., Portland OR 97209 (US)
(72) Inventor: NAIGAMWALLA, Darius, Portland, Oregon 97209 (US); SELF, Fahti, Portland, Oregon 97209 (US); LALLI, Maria, Portland, Oregon 97209 (US); VELLA, Ethan, Portland, Oregon 97209 (US); THOMPSON, Chase, Portland, Oregon 97209 (US)
(74) Representative: Potter Clarkson
(86) International application number: PCT/US2016/069045
(87) International publication number: WO 2017/117310

(56) References cited:
- GB-A- 2 459 076
- US-A- 3 332 414
- US-A- 3 332 414
- US-A- 3 815 585
- US-A- 6 048 308
- US-A- 6 048 308
- US-A1- 2009 099 422
- US-A1- 2009 099 422
- US-B1- 6 379 299
- US-B1- 6 379 299

## Description

### BACKGROUND

The present invention relates generally to the field of medical speculum.

A speculum is a medical tool used to provide visualization into a body cavity. Speculums or specula are traditionally used for viewing the vaginal cavity for gynecology patients. The traditional vaginal speculum consists of two bills with a hinge and a handle. The bills are inserted into the body cavity in a closed position and separated by squeezing two pieces of the handle together, or depressing a thumb lever, thereby dilating the vagina and providing visualization and accessibility of the vagina, the cervix, and surrounding areas. Once opened, the speculum can be locked in an open position using a screw based mechanism so an operator (e.g., physician, nurse, mid-wife, etc.) does not need to continue squeezing the pieces of the handle, or depressing the thumb lever, during the inspection. The operator can then proceed with inspecting the vagina, conducting a Pap smear or any other medical procedures that may need to be provided. Exemplary specula are known from US 3333414 A, US 2009/099422 A1, US 6048308 A, US 6379299 B1, GB 2459076 A and US 3815585 A.

Embodiments herein generally relate to improved speculum devices, components of the same, and methods of making and using the same. The devices and components overcome many drawbacks of existing speculum devices. For example, described herein according to some embodiments are speculum devices that provide a more comfortable and easily manipulated handle design, decreasing the repetitive stress injuries that routinely occur with providers, while also reducing discomfort for the patient.

### SUMMARY OF THE INVENTION

The double bill design of speculum devices has been in use since the 1800s and not many changes have been made to the original design. The biggest changes that have been seen with the double bill design are the addition of plastic as a speculum material, and the addition of internal lighting on some models of the plastic speculum so that the operator does not have to rely on external lighting to get a clear view of the vagina and the cervix. About 15 years ago, an inflatable speculum was developed, but failed to gain any traction in the market and was quickly discontinued.

In the traditional design, speculums include a handle portion and a body portion positioned at substantially 90 degrees relative to one another. In this configuration, insertion into the vagina and maintenance in that position may be difficult and uncomfortable for the practitioner and the patient.

Furthermore, traditional designs incorporate two handle portions to be squeezed together, or a lever to be depressed, to expand the body portion of the speculum. This, too, is difficult for the practitioner with time, due to the ergonomic issues of repeated action, often times multiple times a day. And finally, to hold the speculum in the opened position, speculums of the traditional metal design incorporate a screw and locking nut apparatus wherein once the desired expansion is achieved, the practitioner locks it in by screwing the nut along the shaft until it locks the speculum in place. This is problematic because it requires the practitioner to use both hands to lock in the opened position.

Speculums are traditionally made of metal, though some made with disposable plastic have been increasing in use. When the speculum is made of metal, it can feel cold upon entry, especially in comparison to the internal temperatures of the body, providing discomfort for the patient during the procedure, resulting in the patient tensing up and making the procedure more painful. However, even when made of plastic, the design of the speculum may be generally the same, but for some differences that may exist in the locking mechanisms, wall thickness, and consistencies between the types of plastic.

As noted above, embodiments herein generally related to improved speculum devices, components of the same, and method of making and using the same. The devices and components overcome many drawbacks of existing speculum devices. For example, described herein according to embodiments are speculum devices that minimize the discomfort for the operator.

In one embodiment there is a speculum that includes a body portion comprising a handle configured to be grasped by a user of the speculum, a lower bill, and a transition portion between the handle and the lower bill. The speculum further includes an upper bill rotatably coupled to the lower bill at the transition portion, allowing the upper bill to move relative to the lower bill to move the speculum into an open position, a window frame coupled to the upper bill defining a viewing window, and an actuation mechanism coupled to the upper bill to cause separation of the upper bill from the lower bill. The transition portion creates an angle greater than 90 degrees between the handle and the lower bill. In some embodiments, the angle is in the range of 100 degrees to 180 degrees.

The speculum further includes a locking mechanism configured to hold the speculum in the open position by preventing movement of the upper bill relative to the lower bill when the locking mechanism is engaged. The locking mechanism includes a locking strip having an engagement element, a pawl for interacting with the engagement element which, when interacting, prevents movement of the locking strip in at least one direction relative to the pawl, and a lock switch configured to be actuated to cause interaction of the pawl with the engagement element. In some embodiments, the engagement element is an aperture in the locking strip configured to receive a portion of the pawl which, when interacting, prevents movement of the locking strip relative to the pawl. In some embodiments, the engagement element is a tooth extending from the locking strip configured to interact with a portion of the pawl which, when interacting, prevents movement of the locking strip in one direction relative to the pawl.

In some embodiments, the actuation mechanism is a thumb tab coupled to the window frame. In some embodiments, the speculum further includes an illumination source. In some embodiments, the illumination source is a lighting module having a housing comprising a front plate and a back plate, a lighting element, a power source, and an activation mechanism for providing power to the lighting element prior to use. In some embodiments, the lighting element is an LED. In some embodiments, the power source is a battery. In some embodiments, the activation mechanism is a pull tab provided between a first and a second battery to prevent discharge of the batteries, and wherein when the pull tab is removed, the first and the second battery become electrically coupled and provide power to the lighting element.

In some embodiments, all edges and shape transitions on an outer surface of the handle are rounded. In some embodiments, the speculum further includes a gripping portion. In some embodiments, the gripping portion includes at least a portion made of a different material than a material of the speculum. In some embodiments, the gripping portion includes an overmold placed over the handle. The overmold may be silicone or other similar material.

In another arrangement, there is a locking mechanism for a speculum having a handle, a first bill, and a second bill, the locking mechanism includes a locking strip having an engagement element, the locking strip coupled to the first bill and configured to move in unity with the first bill, a pawl for interacting with the engagement element which, when interacting, prevents movement of the locking strip in at least one direction relative to the pawl, thereby preventing movement of the first bill relative to the second bill, and a lock switch configured to be actuated to cause interaction of the pawl with the engagement element and prevent movement of the locking strip in at least one direction relative to the pawl.

In some arrangements, the engagement element is an aperture in the locking strip configured to receive a portion of the pawl which, when interacting, prevents movement of the locking strip relative to the pawl. In some arrangements, the engagement element is a tooth extending from the locking strip configured to interact with a portion of the pawl which, when interacting, prevents movement of the locking strip in one direction relative to the pawl.

In yet another arrangement, there is a lighting module for a speculum having a housing comprising a front plate and a back plate, a lighting element, a power source; and an activation mechanism for providing power to the lighting element prior to use. In some arrangements, the lighting element is an LED. In some arrangements, the power source is a battery. In some arrangements, the activation mechanism is a pull tab provided between a first and a second battery to prevent discharge of the batteries, and wherein when the pull tab is removed, the first and the second battery become electrically coupled and provide power to the lighting element.

There is a method of performing a medical procedure on a female, comprising, providing a speculum according to any of the embodiments described above; inserting the speculum into the vagina of a female patient; and performing the medical procedure.

There is another method of reducing hand fatigue or injury in a medical professional using a speculum, comprising, providing a speculum according to any of the embodiments described above to the professional for use in a female medical procedure.

### BRIEF DESCRIPTION OF THE DRAWINGS

FIG. 1 is a side view of a speculum according to an exemplary embodiment.
FIGS. 2A-2B are end views of various embodiments of the bills of the speculum of FIG. 1.
FIG. 2C is a cross sectional view of an embodiment of the bills of the speculum of FIG. 1.
FIG. 3 is a rear perspective view of the speculum of FIG. 1.
FIG. 4 is a front perspective view of the speculum of FIG. 1.
FIG. 5A is a cross sectional view of the speculum of FIG. 1.
FIG. 5B is a rear perspective view of a locking mechanism of the speculum of FIG. 1.
FIG. 6 is an inside perspective view of a portion of a handle of the speculum of FIG. 1.
FIG. 7 is a perspective view of a speculum having a lighting module.
FIG. 8 is an exploded view of the lighting module of FIG. 7.
FIG. 9 is a perspective view of a back plate of the lighting module of FIG. 7.
FIG. 10A depicts a front perspective view of a first alternative actuation feature for expanding the expandable body of a speculum.
FIG. 10B depicts a side view of the first alternative actuation feature for expanding the expandable body of a speculum.
FIG. 11A depicts a side view of a second alternative actuation feature for expanding the expandable body of a speculum.
FIG. 11B depicts a front perspective view of the second alternative actuation feature for expanding the expandable body of a speculum.
FIG. 11C depicts a side view of a third alternative actuation feature for expanding the expandable body of a speculum.
FIG. 11D depicts a front perspective view of the third alternative actuation feature for expanding the expandable body of a speculum.
FIG. 12 depicts a front perspective view of the fourth alternative actuation feature for expanding the expandable body of a speculum.

### DETAILED DESCRIPTION

In the following detailed description, reference is made to the accompanying drawings, which form a part of the present disclosure. In the drawings, similar symbols typically identify similar components, unless context dictates otherwise. The illustrative embodiments described in the detailed description, drawings, and claims are not meant to be limiting. The detailed description is intended as a description of exemplary embodiments and is not intended to represent the only embodiments which may be practiced. The term "exemplary," as used herein, means "serving as an example, instance, or illustration," and should not necessarily be construed as preferred or advantageous over other embodiments. Other embodiments may be utilized, and other changes may be made, without departing from the scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and form part of this disclosure.

Referring to the figure generally, a speculum system is shown. The speculum is an updated design relative to the antiquated, traditional two-bill design. The speculum has an ergonomic handle for increased comfort during use. Without being limited thereto, in some embodiments, the handles described herein can have one or more features selected from a greater angle between the grip or the handle portion and the bills of the speculum, a textured grip on the handle, the size of the handle, etc. As one example, the larger angle can be beneficial so that the handle does not interfere or contact patient as readily while being inserted, while inserted, or when removed and also can be more comfortable for the user by providing a neutral position for the wrist. As another example, the ergonomic handle with a textured grip can provide for improved precision during use, for example, due to ergonomic touch points. The improvements can minimize hand fatigue or other stresses to the hand including carpel tunnel problems for the user. In addition, an improved locking mechanism can allow the speculum to be used with one hand, as opposed to locking mechanisms that require the use of two hands. In some embodiments, the profile of the speculum, for example the bill portion, may be narrower than traditional speculum while still maintaining an equal or greater level of usability. In some embodiments, the speculum may be used with a sleeve that can be placed over the bill portion to provide side wall support. The sleeve may be disposable, reusable, lubricated, or include a therapeutic agent.

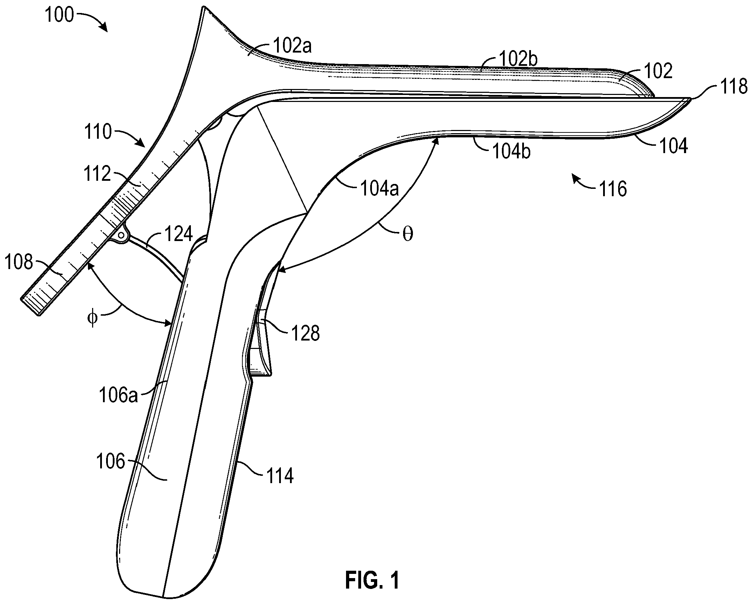

FIG. 1 depicts a speculum 100 having an upper bill 102, a lower bill 104, a handle 106, a viewing window 110, and an actuation mechanism. The speculum 100 shown in FIG. 1 has an updated ergonomic design in accordance with certain embodiments of an updated speculum. The speculum 100 may be made of any sturdy biomaterial including metals and plastics. The lower bill 104 and the handle 106 are formed together as a unitary body portion. The upper bill 102 is rotatably coupled to a transition portion 104a between the lower bill 104 and the handle 106 such that the upper bill is movable between an open position and a closed position when the actuation mechanism, such as the actuation tab 108 is manipulated. When the bills 102 and 104 are in the closed position, the speculum 100 can be inserted in a patient's vagina. The user places the end 118 in line with an opening of the vagina and applies a force along a longitudinal axis of the bills 102 and 104 to push the bills 102 and 104 into the vagina. The user may position the speculum 100 at a depth of the vagina to provide a clear view of the cervix when the bills 102 and 104 of the speculum are opened. The speculum 100 may be inserted with the handle portion in a substantially vertical direction, as in the orientation of FIG. 1. Alternatively, the speculum 100 may be inserted with the handle portion in a substantially horizontal direction and rotated to a vertical position once inside the vagina. The speculum may also be inserted with the bills at a downward 45 degree angle for patient comfort. The speculum 100 may be inserted so that the end 118 of the bills 102 and 104 are located below the cervix. Once the bills 102 and 104 are separated, the cervix may then fall into the viewing window created by the separation of the bills 102 and 104. Alternatively, the speculum may need to be shifted in different directions in order to view the cervix properly.

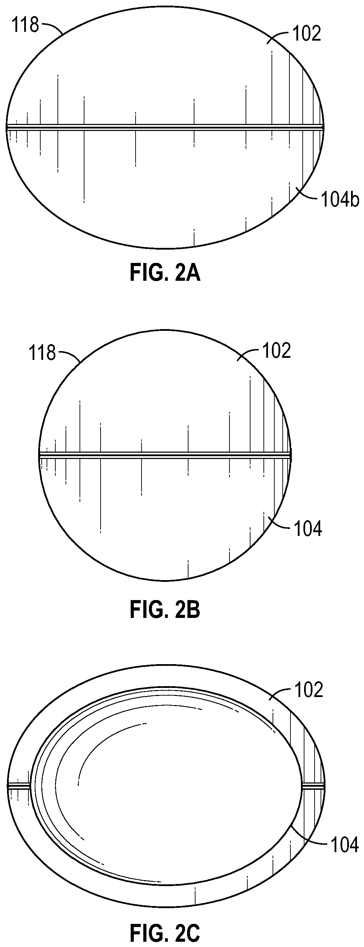

The bills 102 and 104 each comprise a transition portion 102a, 104a and a main bill portion 102b, 104b. The main bill portions 102b, 104b form an elongated portion 116 of the speculum 100. Various embodiments of the elongated portion 116 are shown in the end views of FIGS. 2A-2B. The elongated portion 116 may have a width that is larger than a height of the elongated portion, creating an oblong shape, as seen in FIG. 2A. Alternatively, the height and width of the elongated portion 116 may be configured in such a way that when the bills 102 and 104 are closed, a circular cross section is formed, as seen in FIG. 2B. At an end 118 of the elongated portion 116, away from the handle 106, the bills 102 and 104 may be rounded. A rounded end 118 may provide more comfort to a patient whom will be receiving the speculum 100 in a cavity. The bills 102 and 104 may also be configured such that when in the closed position, the end 118 of each bill 102 and 104 may abut one another without any gaps. In other embodiments, the top bill 102 or the bottom bill 104 may be longer than the other (see FIG. 1) to create a gap when the bills are closed, preventing tissue from becoming lodged in between the bills 102 and 104 during insertion or closing of the bills. The bills 102 and 104 may have a semi-circular cross section along a length of the bill 102 and 104, where the flat portions of the semi-circles are moved together when the bills 102 and 104 are in a closed position, as seen in FIG. 2B. In these embodiments, an outer edge and an inner edge of the bills are preferably each curved such that when the bills 102 and 104 are in the closed position, a cross section of the bills 102 and 104 forms a cylinder shape, as seen in FIG. 2C.

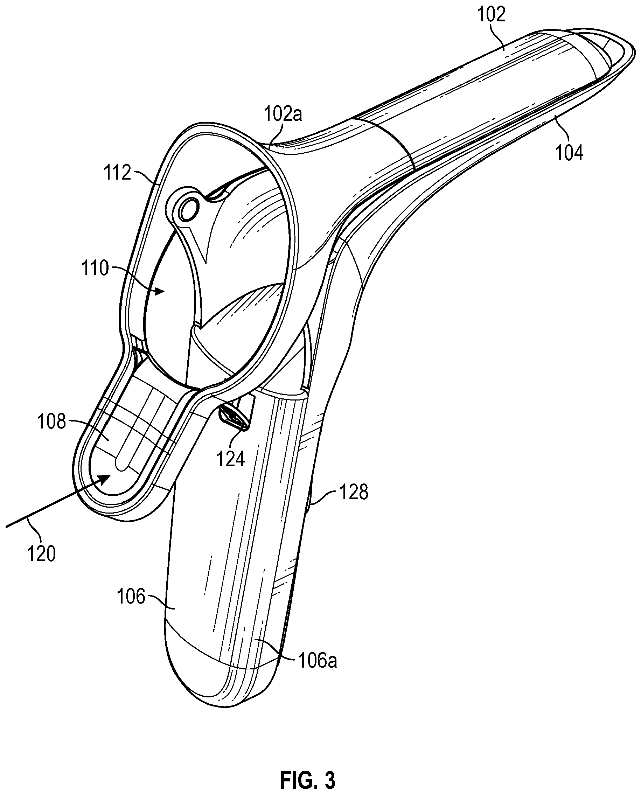

As shown in FIG. 3, speculum 100 includes a window 110 defined by a window frame 112. The window frame 112 is unitary with the upper bill 102 and extends from a proximal portion of the transition portion 102a of upper bill 102. The transition portion 102a is slightly curved such that a top portion of the window frame 112 is positioned higher than the top of upper bill 102. Referring back to FIG. 1, from the transition portion 102a, the window frame 112 extends at an angle Φ away from handle 106. In some embodiments, the angle Φ is greater than 25 degrees but less than 40 degrees, or any degree value or sub range of degrees therein. In some embodiments, the angle may be between 30 and 35 degrees. In some embodiments the angle Φ is about 25, 26, 27, 28, 29, 30, 31, 32, 33, 34, 35, 36, 37, 38, 39, 40, 41, 42, 43, 44, or 45 degrees. In some embodiments, the angle Φ may define the extent to which upper bill 102 is able to separate from lower bill 104. When window frame 112 contacts handle 106 as force 120 is applied (described below), the contact will limit the range of motion of the upper bill 102 relative to lower bill 104. In other embodiments, a limit to the range of motion is provided in another way.

Still referring to FIG. 3, the speculum 100 has an actuation mechanism, shown as actuation tab 108, to cause separation of bills 102 and 104. In this embodiment, actuation tab 108 is formed unitary with the upper bill 102 at a lower portion of the window frame 112. A force 120 is applied, preferably by a thumb of the user, to the actuation tab 108 which causes the bills 102 and 104 to separate. When force 120 is applied to the actuation mechanism, the opening created by the separation of the bills 102 and 104 is caused by the upper bill 102 moving relative to the lower bill 104 and handle 106. For example, the force 120 applied on the actuation mechanism causes upper bill 102 to move, while lower bill 104 remains stationary. In other embodiments, the opening may be caused by both bills 102 and 104 moving, or lower bill 104 moving. Upper bill 102 is preferably coupled to the transition portion 104a by a hinge such that force 120 on the actuation mechanism causes a rotational movement of bill 102 about the hinge, separating the ends of bills 102 and 104.

In the embodiment shown, tab 108 has a concave surface to cooperate with the shape of the thumb of the user and provide a more stable area for the thumb to apply force 120. In some embodiments, the tab 108 is a separate element that is coupled to the window frame 112. In other embodiments, the tab may be formed unitary with the window frame. The applied force 120 should not need to be a substantial force. As described above, in some embodiments, a distance the actuation mechanism moves correlates to a distance the bills 102 and 104 separate. In this regard, the user would be able to modify the specific distance between the bills 102 and 104 for each patient. The bills 102 and 104 may open in a continuous fashion when force 120 is continuously applied to the actuation mechanism. The user may have to hold the actuation mechanism in place in order to maintain the distance between the bills 102 and 104. Alternatively, the speculum 100 may have a locking mechanism, such that the bills 102 and 104 can be locked in an open position. In this regard, the user would not have to maintain pressure on the actuation mechanism to maintain the distance between bills 102 and 104.

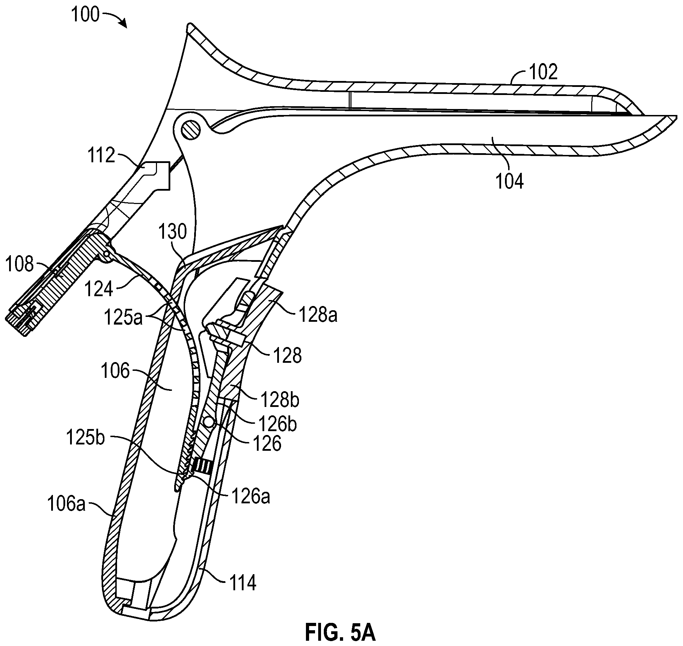

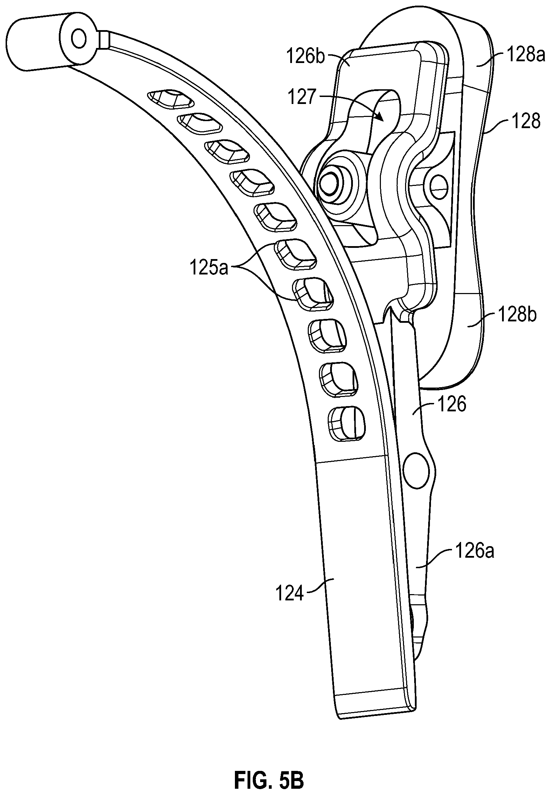

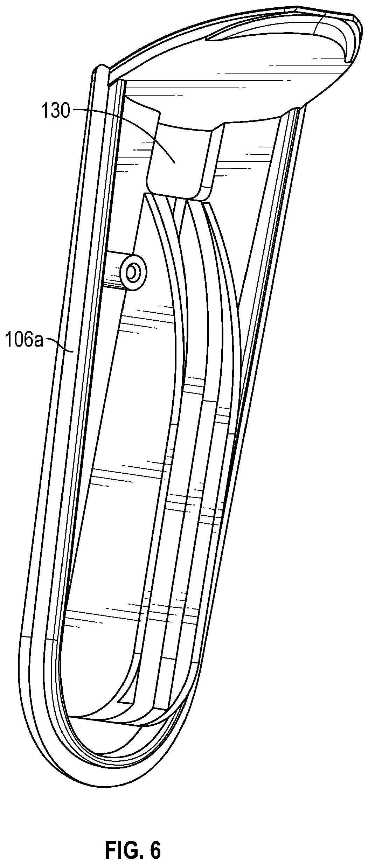

For example, referring to the cross-sectional view of FIG. 5A and to FIG. 5B, speculum 100 has a locking mechanism which includes a locking strip 124, a pawl 126, and lock switch 128. Locking strip 124 is coupled to tab 108 or window frame 112. In some embodiments, the locking strip 124 and tab 108 are formed together as a unitary element. In other embodiments, the locking strip 124 and tab 108 are separable coupled together during construction of the speculum 100. As force 120 is applied to thumb tab 108, thereby opening bills 102, 104, locking strip 124 moves through a window 130 in a back cover 106a of handle 106 (shown in FIG. 6). The locking strip 124 may include a plurality of teeth 125b. As locking strip 124 moves with the opening of upper bill 102 from lower bill 104, the teeth 125b of the locking strip 124 move into position to engage with end portion 126a of pawl 126. When end portions 126a of pawl 126 engages in one of the plurality of teeth 125b in the locking strip 124, the locking strip 124 is prevented from moving in a direction such that the upper bill 102 is prevented from closing, and therefore, the bills 102, 104 are locked in the open position. When the lock switch 128 is in a locked position, the teeth 125b will allow movement of the locking strip 124 relative to the pawl 126 in a direction corresponding with the opening of the bills 102, 104, and will prevent movement of the locking strip 124 relative to the pawl 126 in an opposite direction. Locking strip 124 further has a plurality of openings 125a therethrough that are configured to provide flexibility to the locking strip 124.

According to the embodiment shown, the locking mechanism further includes lock switch 128. The pawl 126 includes an aperture 127, through which a connection mechanism of the lock switch 128 passes through to couple the lock switch 128 inside the handle. As shown in FIG. 5A, when the bottom portion 128b of lock switch 128 is toggled, or pressed in, the end portion 126a of pawl 126 is pressed towards the locking strip 124. The end portion 126a may be spring biased towards the lock strip 124. When aligned with one of the plurality of openings 125a, the end portion 126a engages the locking strip 124. In this way, pressing the bottom portion 128b of the lock switch 128 causes the locking strip 124 to be held in place relative to the pawl 126 and prevents the bills 102, 104 from closing. When the top portion 128a of lock switch is toggled, or pressed in, the top portion 126b of pawl 126 is caused to be pressed towards the locking strip 124. Accordingly, the engagement between the pawl 126 and the locking strip 124 is released and the locking strip is able to move relative to the pawl 126. As a result, upper bill 102 is free to move relative to lower bill 104, and the speculum 100 is no longer locked. In other embodiments, the locking mechanism could have an alternative configuration, wherein the toggling of the lock switch functions in opposition, for example, pressing the top portion 128a of the lock switch 128 locks the mechanism, and pressing the bottom portion 128b unlocks the mechanism.

The locking mechanism shown in FIG. 5 is configured such that when the lock switch 128 is in a locked position (e.g., the bottom portion 128b is toggled), the bills 102, 104 are still able to open, but the movement will be incremented due to the engagement of the pawl with the teeth. In this way, there will be resistance and noise associated with opening the bills 102, 104. As mentioned above, in the locked position, the bills are substantially prevented from closing unless a force in the opposite direction is applied. In order to open the bills freely (i.e., limited noise or resistance) and/or to close the bills, the lock switch should be moved to the unlocked position (e.g., top portion 128a toggled). The ability to open the speculum with minimal resistance and noise is an improvement over the presently used speculum designs.

Once the user has completed the inspection of the vaginal cavity and cervix, the bills 102 and 104 may be returned to the closed or mostly closed position prior to or during removal the speculum 100 from the patient. To return the bills 102 and 104 to the closed position, the user may release the force 120 from the actuation mechanism. Removing force 120 would work to close the bills 102 and 104 if the force 120 needed to be applied for the length of the procedure to maintain the viewing window. However, if the actuation mechanism locks into place when force 120 is applied, a user will unlock the speculum to close the bills 102 and 104. For example, using the locking mechanism depicted in FIG. 5, a user would press the lower portion 128b of the lock switch 128 to disengage the pawl 126 from the locking strip 124, and therefore, unlock the speculum 100 to close the bills 102 and 104.

In alternative embodiments, a second force may be applied to the actuation mechanism to close the bills 102 and 104. The second force may be applied in a direction opposite of force 120. The second force should be small enough so as to be easy to apply with an upward movement of the thumb of the user. The user should be able to apply the second force while maintaining control of the speculum 100. Alternatively, the actuation mechanism may be released by applying a second force 120 to the actuation mechanism to move the actuation mechanism past the locking position, releasing the lock and closing the bills 102 and 104.

Once the bills 102 and 104 of the speculum 100 are closed, the speculum 100 can be removed from the patient. When closing the bills 102 and 104, the user should take caution to ensure no vaginal tissue is between the bills 102 and 104 such that the tissue may become pinched when closed. The speculum 100 should then be pulled along an axis along the length of bills 102 and 104 to easily remove the bills 102 and 104 from the patient.

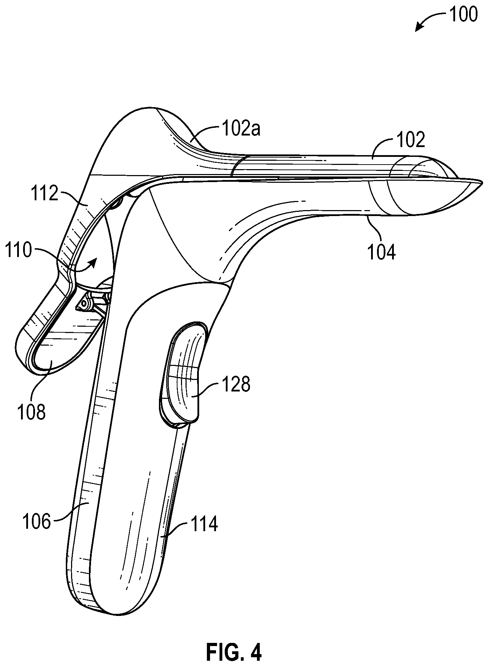

The actuation mechanism should be located at a position such that the user is able to easily reach the actuation mechanism with a thumb when the rest of a hand of the user is holding handle 106. In the embodiments of FIGS. 1 and 3-5, the actuation mechanism is located on a proximal end of the upper bill 102, coupled or unitary with the window frame 112. In some alternative embodiments of a speculum, not depicted in the figures, an actuation mechanism may be located on the handle 106 on a user side (i.e., the side of the handle opposite the bills 102 and 104). The actuation mechanism may be located along a center axis of the handle 106. In this regard, the speculum 100 could be used in both a right hand or a left hand. In the embodiments shown herein, the actuation tab 108 is aligned with a center axis of the handle 106. Alternatively, the actuation mechanism may be offset from the center axis of the handle 106. In this regard, the location of the actuation mechanism may cause the speculum 100 to be suited specifically for either a right hand or a left hand. If the actuation mechanism is located on the user side of the handle 106 opposite the bills 102 and 104, the user side of the handle 106 may be substantially flat to facilitate coupling of the actuation mechanism and the handle 106. In another embodiment, the actuation mechanism lever 108 may be located on an edge of the handle 106. In this regard, the speculum 100 is configured to be used by either a right hand or a left hand depending on which edge of the handle 106 is coupled to the actuation mechanism.

Referring to FIGS. 1 and 3-5, it is shown that the speculum 100 employs an obtuse angle θ between the bill 104 and the handle 106, which is more comfortable for both the practitioner and the patient during use. The transition portion 104a creates the angle θ between the lower bill 104 and the handle 106. In some embodiments, the angle θ is greater than 90 degrees but less than 180 degrees, or any degree value or sub range of degrees therein. In some embodiments, the angle may be between 100 and 150 degrees. In some embodiments, the angle θ is about 95, 100, 105, 110, 115, 120, 125, 125, 130, 135, 140, 145, 150, 155, 160, 165, 170 or 180 degrees. The angle θ provides a more comfortable angle for the user to insert the speculum 100 into the vagina of the patient. In addition, the angle θ provides more room for the hand of the user when inserting the speculum 100. This reduces the risk of the hand of the user coming into contact with the patient when inserting the speculum 100, which can be uncomfortable and awkward for the patient. This also can reduce the possibility or degree of interference during use due to contacting the body of the patient, as well as the examination table or bed that the patient may be seated or lying upon, for example. The angle θ also provides a more comfortable angle for a wrist of the user during the procedure. This reduces the risk of injury or strain to the hand or wrist of the user, especially when the user completes multiple procedures in a single day, or day after day.

The handle 106 may also be rounded, lacking sharp corners and edges, and have a circular cross section to provide a more comfortable and natural fit in the hand of the user. Alternatively, a patient side of the handle 106 may be rounded while a user side may be flat. This configuration may provide the user a flat portion to rest a thumb of the user during the procedure. In another embodiment, both the user and patient sides are rounded, but with different curvatures such that the cross section is more oblong than circular. The handle 106 may include a grip 114 for comfort and ergonomic benefit. The grip 114 may be of a material that provides more traction for the hand of the user, for example, the grip 114 may be an overmold placed over a portion of the handle. The overmold may be silicone or other similar material. In some embodiments, the grip 114 is textured to provide more traction for the hand of the user. For example, the grip 114 may include bumps, dimples, and/or other texturizing elements, and a rounded or formed body for comfort and ergonomic benefit. The grip texture can include any suitable feature to permit gripping of the handle. For example, the texture can include raised elements or protrusions such as bumps, ridges (e.g., vertical or horizontal, straight or waved, etc.), and/or indentations or holes, which can be in the shape of pinholes, grooves, channels, etc. The grip 114 may allow the user to apply less force with the hand of the user in order to hold the speculum 100. By allowing the user to use less force to hold the speculum, the user may become less fatigued when performing the procedure. In addition, when less force is needed, the user may experience less cramps, strains and/or injuries caused by using the speculum 100. The grip 114 may extend the length of the handle 106. In some embodiments, the grip 114 only extends a portion of the length of the handle 106. In some embodiments, the grip 114 may be located only on the user or patient side of the handle 106. In other embodiments, the grip 114 may be located on both the user and patient side of the handle 106 in separate pieces. In another embodiment, the grip 114 wraps around the circumference of the handle 106. In yet another embodiment, the grip 114 is a plurality of pieces spaced along the handle 106. In another embodiment, the grip 114 is made of grooves located along the handle 106, where the grooves align with where fingers of the user would be located when holding the handle 106. The grip 114 may also provide for cushioning the practitioner's hold on the handle, and/or for a more secure grip on the device.

The handle 106 may be made of metal and/or plastic, including, but not limited to, titanium, aluminum, stainless steel, acrylic, polyethylene, polyester, polyethyleneaphthlate, polystyrene, polyvinylchloride, polyethersulfone, polyetherimide, polycarbonate, polysulfone, polyetheretherketone, polyphenylsulfone, and polymethyl methacrylate. The handle 106 may be made of a material that can be sterilized. The handle 106 may be made of material that is biocompatible. The handle 106 may be made using a variety of techniques including, but not limited to, injection molding, extrusion, machining, blow molding, rotational molding, compression molding, transfer molding, stamping, and casting.

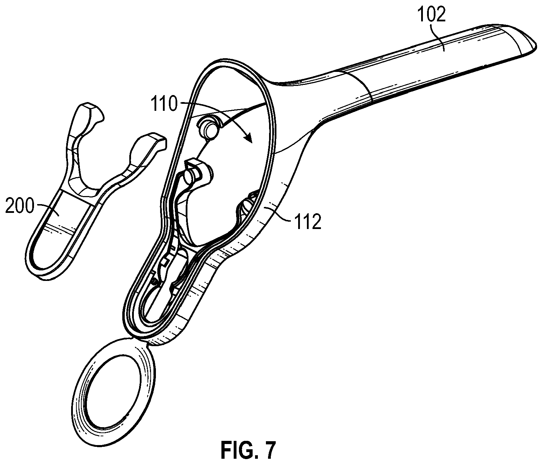

FIG. 7 depicts another embodiment of the speculum 100 having a lighting module 200. In this embodiment, the lighting module 200 is Y-shaped, having a base section 200a and two extensions 200b. The lighting module 200 is positioned in the window 110 and held in place by engagement with the window frame 112. For example, the lighting module 200 may have a snap fit relationship with the window frame 112. In other embodiments, the lighting module may be attached to the window frame 112 using fixation devices (i.e., screws, nails, etc) or adhesive (i.e., tape or glue). The base section 200a is configured to replace the thumb tab 108, but function in the same way as thumb tab 108 for providing an actuating mechanism for opening the bills 102 and 104. The lighting module is configured to illuminate the cavity in which the speculum 100 is placed in order to allow better visualization of the cavity and execution of procedures in the cavity. The lighting module 200 provides one or more illumination elements, such as LEDs 204. The configuration of the lighting module 200 provides illumination elements at or near the perimeter of the window 110 at window frame 112, so as not to obstruct the view of the user through the window 110. In the embodiment shown, an illumination element is positioned on each side of the window frame 112. The illumination element(s) may alternatively be positioned near any part of the window frame 112. In some embodiments, one or more of the illumination elements may be positioned to interface with one or more light pipes which extends through at least a portion of the length of the top bill 102, thereby directing light through the bill 102.

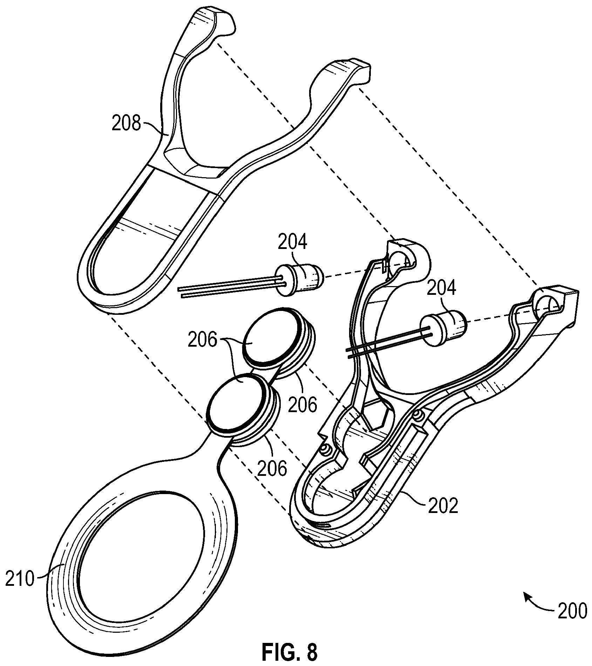

As shown in FIG. 8, lighting module 200 includes a back plate 202, an LED 204, a resistor (not shown), a battery 206, a front plate 208, and a pull tab 210. The back plate 202 and the front plate 208 form a housing to carry the elements of the lighting module 200. In some embodiments, the lighting module 200 includes more than one LED 204. In some preferred embodiments, the lighting module 200 includes two LEDs 204. The LED may be any color suitable for providing lighting to the cavity. In preferred embodiments, the LED is clear or white. Various sizes of LEDs 204 can be used in lighting module 200, such as 1 mm, 3mm, 5mm, and 10mm LEDs. In order to limit current into the LED 204, so as not to burn out the LED 204, a resistor (not shown) is used in series with the LED 204 in the lighting module 200. The value of the resistor needed depends on the forward current and the forward voltage specifications of the LED 204 (variable), and on the power source voltage.

Lighting module 200 also includes a power source, such as one or more batteries. In the embodiment shown, two button cell batteries 206 are used to power each LED 204 electrically coupled thereto (through in line resistors). Each battery 206 preferably has a voltage of about 3.0V, but batteries having other voltages may also be used. The lighting module also includes an activation mechanism which can be manipulated to cause the LEDs to go from an "off' state to an "on" state. In this embodiment, the activation mechanism is a pull tab 210 positioned between the batteries, thereby preventing discharge of the batteries and keeping the LEDs "off." The pull tab 210 is configured to be pulled by the user to dislodge the pull tab 210 from between the batteries 206, thereby allowing the flow of current and providing power to the LEDs. In this embodiment, there is no mechanism for powering the LEDs off after they have been turned on. In some embodiments, the same activation mechanism may be used to power off the LEDs or there may be a second mechanism for powering off the LEDs.

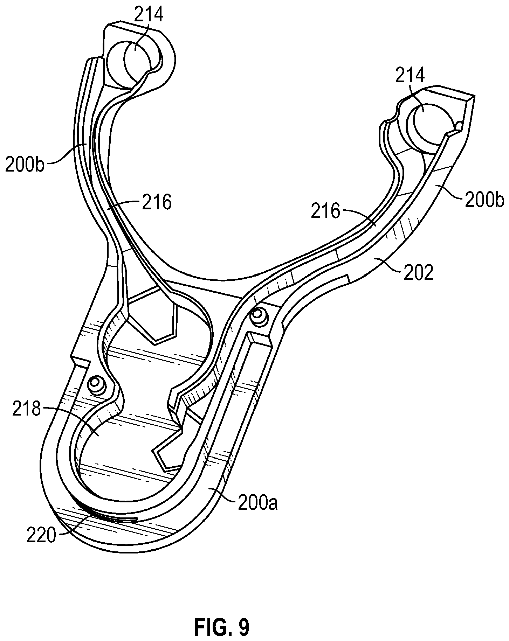

FIG. 9 shows a perspective view of the back plate 202, which is configured to receive and hold the elements of the lighting module 200 therein. As shown, the back plate has an LED aperture 214 at the top of each extension 200b for each LED 204. An LED 204 is positioned in each aperture 214 and can illuminate through into the cavity. Along each extension 200b is a passage 216 along which the legs of each LED 204 and the inline resistors are positioned, to connect with the batteries 206. One passage 216 leads to one pair of batteries 206 while the other passage 216 leads to the other pair of batteries, as shown in the figure. The back plate 202 further includes a battery receptacle 218 for receiving the batteries 206. A slot 220 is located at a bottom edge 222 of the back plate, through which the pull tab 210 extends before it is removed by the user.

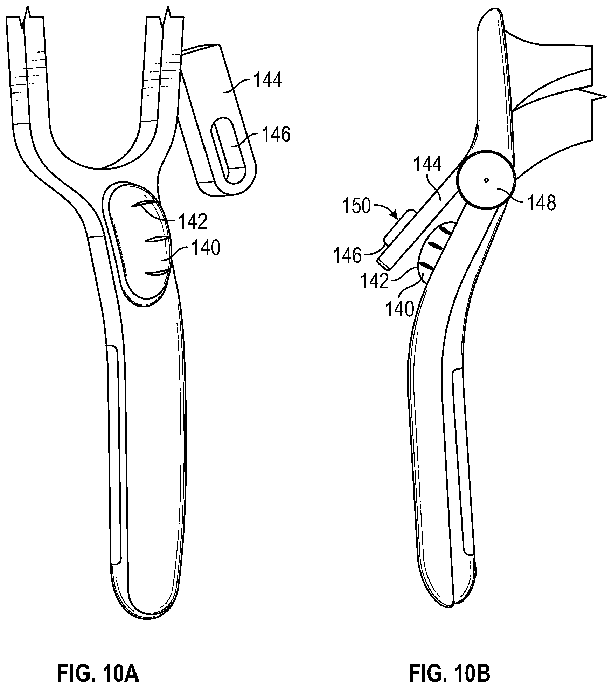

FIGS. 10-12 depict alternative embodiments of a speculum not forming part of the present invention. It is contemplated that any of the features, or combinations thereof, recited above may be used with any other features described in other embodiments, including the features described with reference to FIGS. 10-12. Specifically, FIGS. 10-12 relate to various alternative embodiments of the actuation feature for opening, locking, and/or closing the bills 102 and 104 of the speculum 100.

FIGS. 10A and 10B depict a scroll and press mechanism, in accordance with an exemplary embodiment. With the scroll and press mechanism, the distance between the bills 102 and 104 can be set while expanding or prior to expanding the speculum within the patient.

The scroll and press mechanism includes a scroll wheel 140. The scroll wheel 140 may be embedded into the handle 106 such that only a portion of the scroll wheel 140 is visible and accessible to the user. The scroll wheel 140 can be rotated in two directions. When the scroll wheel 140 is rotated in a first direction, the diameter of the speculum 100 increases. The increase in diameter moves the bills 102 and 104 further away from one another while in the closed position. When the scroll wheel 140 is rotated in a second direction, the diameter of the speculum 100 decreases. The decrease in diameter moves the bills 102 and 104 closer to one another. The user may obtain a better view of the cervix by properly adjusting the diameter of the speculum 100 to the diameter of the cervix in the vagina. The scroll wheel 140 may also include ridges or grooves 142 to allow the user to rotate the scroll wheel 140 more easily. In some embodiments, the ridges or grooves 142 provide an indication of the diameter the speculum. The scroll wheel 140 may provide fluid increases and decreases in diameter as the scroll wheel 140 is rotated. Alternatively, the scroll wheel 140 may provide fixed increases and decreases in diameter as the scroll wheel 140 is rotated. For example, a full 360 degree rotation may be needed to increase or decrease the diameter by a set amount. The user may have the ability to lock the scroll wheel 140 into place when an appropriate diameter is selected. Subsequently, the user may be able to unlock the scroll wheel 140 when the examination has concluded.

The scroll wheel 140 may be located on the handle 106. The scroll wheel 140 should be located in such a way that the user can easily rotate the scroll wheel 140 with a thumb while maintaining a secure grip on the handle 106. The scroll wheel 140 should not interfere with the ability of the user to move and adjust the placement of the speculum 100 into the vagina of the patient.

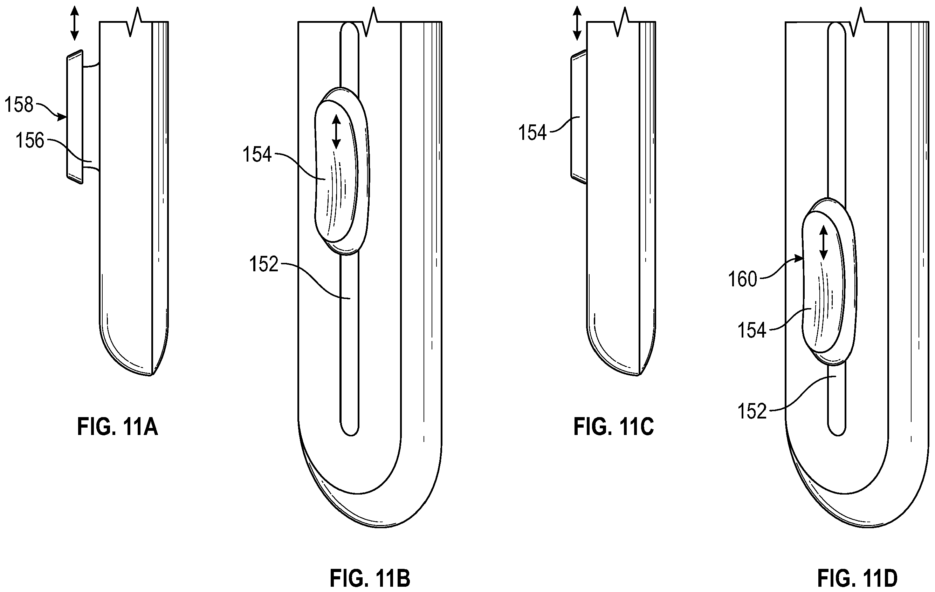

FIGS. 11A-11D depicts an alternative actuation or locking feature in accordance with certain embodiments of the speculum, not forming part of the invention. The actuation feature of FIGS. 11A-4D use a squeeze and slide mechanism to open and/or lock the speculum.

The squeeze and slide mechanism includes a groove 152. The groove 152 is an elongated opening that provides a track for a slider 154. The groove 152 may be rounded at each end. The groove 152 may be located on the handle 106 of the speculum 100. The groove 152 has a length that is shorter than a length of the handle 106. Inside the groove 152 is a mechanism that opens and closes the bills, basedon the movement of the slide 154. In other embodiments, an actuation mechanism as described above with respect to speculum 100 may be used, and the slide 154 is used to move between a locked and an unlocked state.

The slider 154 has an elongated body and can be moved in two directions along the groove 152. Movement in a first direction causes the bills 102 and 104 to separate, increasing the diameter of the opening created by the speculum 100. Movement in a second direction causes the bills 102 and 104 to close, decreasing the diameter of the opening created by the bills 102 and 104. In other embodiments, where the bills are opened by a separate actuation mechanism, movement in the first direction causes the speculum to lock and movement in the second direction causes the speculum to unlock, or vice versa. The slider 154 may have a rounded, elongated body to facilitate the placement of the thumb on the slider 154. The slider 154 should be located in such a way that the user can easily apply a force to the slider 154 with the thumb while maintaining a secure grip on the handle 106. The slider 154 should not interfere with the ability of the user to move and adjust the placement of the speculum 100 into the vagina of the patient.

In one embodiment, the slider 154 includes a spacer 156 to raise the slider 154 out of the groove 152. In this embodiment, the user applies a force 158 to the slider 154. The force 158 causes the slider 154 to abut the groove 152 and activate the intemalmechanism. The user can slide the slider 154 up and down along groove 152 while force 158 is applied to adjust the diameter of the opening created by the bills 102 and 104 and/or move between a locked and unlocked position. Once force 158 is removed from slider 154, spacer 156 is visible again. The slider 154 is locked into place when no force is applied.

In other embodiments, the slider 154 remains flush with the groove 152. In this embodiment, the user applies a force 160 to the slider 154, to slide the slider 154 up and down along groove 152 to adjust the diameter of the opening created by the bills 102 and 104 and/or move between a locked and unlocked position. Once force 160 is removed from slider 154, the slider 154 is held in place.

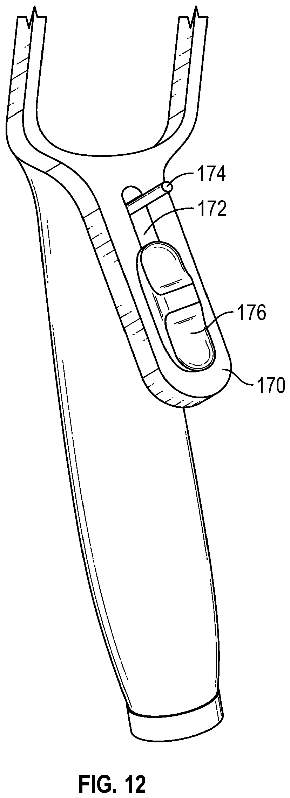

Fig. 12 depicts an alterantive actuation or locking feature in accordance with certain embodiments of the speculum, not forming part of the invention. The actuation feature of Fig. 12 uses a guide and lock mechanismto open and/or lock the speculum.

The mechanism of Fig. 12 includes a base 170, a groove 172, a guiding member 174 and a locking member 176. In some embodiments, the base 170 may be coupled to the actuation mechanism, such as actuation tab 108, of the speculum. In another embodiment, the base 170 may be coupled to the handle 106 of the speculum. The groove 172 may extend through a depth of the base 170. In some embodiments, the groove 172 may extend longitudinally for a length of the base 170. In some embodiments, the groove 172 may extend longitudinally for only a portion of the length of the base 170. The guiding member 174 may be coupled to the speculum on a first end. A second end of the guiding member 174 may extend through the groove 172. The configuration of the guiding member 174 may allow the base 170 to move up and down at an angle keeping the guiding member 174 positioned in the groove 172.

The movement of the guiding member 174 in relation to the base 170 may cause the bills of the speculum to separate. To lock the bills in an open position, the locking member 176 may be moved in a first direction (e.g., in an upward direction), along a longitudinal axis of the base 170. The locking member 176 may use groove 172 as a track to slide. Alternatively, locking member 176 may use a separate aperture to slide. To unlock the bills (i.e., return the bills to a closed position), the locking member 176 may be moved in a second direction (e.g., in a downward direction), along the longitudinal axis of the base 170. The locking member 176 may be shaped to conform to the shape of a thumb or finger of the user. The locking member 176 may also include a ridge to increase the ease of moving the locking member 176. The ridge may be centered on the locking member 176 and orientated perpendicular to the longitudinal axis of the base 170. The locking member 176 may be shaped to conform to the shape of guiding member 174 when in the locked position.

For the practitioner, the features of the present disclosure may reduce fatigue and repetitive stress injury, allow for one-handed opening and locking, allow increased visibility and accessibility, along with many other benefits. For the patients, these features may reduce patient anxiety because they employ quieter mechanisms than the traditional designs and because of the updated look of the lever 108 and handle 106.

Some of the disclosure herein which does not form part of the invention relates to methods of performing obstetric or gynecological procedures utilizing speculum devices having a handle with one or more of the features described herein. Non-limiting examples of such procedures include pelvic exams, pap smears, insemination, IUD insertion/removal. In some embodiments, the methods can include performing a plurality of such procedures in a given period of time, such as an 8 hour or 24 hour period of time, or any sub period of time therein. Other embodiments relate to methods of reducing hand fatigue or repetitive use injury in a user of a device or handle as described herein.

The foregoing description details certain embodiments of the systems, devices, and methods disclosed herein. It will be appreciated, however, that no matter how detailed the foregoing appears in text, the devices and methods can be practiced in many ways. The scope of the disclosure should therefore be construed in accordance with the appended claims.

It will be appreciated by those skilled in the art that various modifications and changes may be made without departing from the scope of the described technology. It will also be appreciated by those of skill in the art that parts included in one embodiment are interchangeable with other embodiments; one or more parts from a depicted embodiment can be included with other depicted embodiments in any combination. For example, any of the various components described herein and/or depicted in the Figures may be combined, interchanged or excluded from other embodiments.

The devices, components, methods and systems described herein can be combined with one or more of the devices, components, methods and systems described in any of U.S. Patent Application entitled "Speculum with Secondary Bills," filed on December 28, 2016 and identified by Atty. Docket No. 112359-0253, U.S. Patent Application entitled "Insertable Sleeve for Speculum and Use Thereof," filed on December 28, 2016 and identified by Atty. Docket No. 112359-0353, and U.S. Patent Application entitled "Sleeve for Speculum and Use Thereof," filed on December 28, 2016 and identified by Atty. Docket No. 112359-0403.

With respect to the use of any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" should be interpreted as "including but not limited to," the terms "comprising" and "having" should, respectively, be interpreted as "comprising at least" and "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an." In general, "a" and/or "an" should be interpreted to mean "at least one" or "one or more"; the same holds true for the use of definite articles used to introduce claim recitations.

Furthermore, in those instances where a convention analogous to "at least one of A, B, and C, etc." is used, in general, such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

The technology disclosed herein has numerous applications and while particular embodiments of the technology have been described in detail, it will be apparent to those skilled in the art that the disclosed embodiments may be modified given the design considerations discussed herein. Therefore, the foregoing description is to be considered exemplary rather than limiting, and the true scope of the invention is that defined in the following claims.

## Claims

1. A speculum (100) comprising:
a body portion comprising a handle (106) configured to be grasped by a user of the speculum (100), a lower bill (104), and a transition portion (104a) between the handle (106) and the lower bill (104);
an upper bill (102) rotatably coupled to the lower bill (104) at the transition portion (104a), allowing the upper bill (102) to move relative to the lower bill (104) to move the speculum (100) into an open position;
a window frame (112) coupled to the upper bill (102) defining a viewing window;
an actuation mechanism coupled to the upper bill (102) to cause separation of the upper bill (102) from the lower bill (104); and
a locking mechanism configured to hold the speculum (100) in the open position by preventing movement of the upper bill (102) toward the lower bill (104) when the locking mechanism is engaged, the locking mechanism comprising:
a locking strip (124) contained within the handle (106), the locking strip (124) having an engagement element;
a pawl (126) for interacting with the engagement element which, when interacting, prevents movement of the locking strip (124) in at least one direction relative to the pawl (126); and
a lock switch (128) configured to be actuated to cause interaction of the pawl (126) with the engagement element;
wherein the transition portion (104a) creates an angle greater than 90 degrees between the handle (106) and the lower bill (104).

2. The speculum (100) of claim 1, wherein the angle is in the range of 100 degrees to 180 degrees.

3. The speculum (100) of claim 1, wherein the engagement element is an aperture (125a) in the locking strip configured to receive a portion of the pawl (126) which, when interacting, constrains movement of the locking strip (124) relative to the pawl (126).

4. The speculum (100) of claim 1, wherein the engagement element is a tooth (125b) extending from the locking strip (124) configured to interact with a portion of the pawl (126) which, when interacting, prevents movement of the locking strip (124) in one direction relative to the pawl (126).

5. The speculum (100) of claim 1, wherein when the locking mechanism is disengaged, the upper bill (102) moves relative to the lower bill (104) without resistance and without sound.

6. The speculum (100) of claim 1, wherein the actuation mechanism is a thumb tab (108) coupled to the window frame.

7. The speculum (100) of claim 1, further comprising an illumination source.

8. The speculum (100) of claim 7, wherein the illumination source is a lighting module (200) comprising:
a housing comprising a front plate (208) and a back plate (202);
a lighting element;
a power source; and
an activation mechanism for providing power to the lighting element prior to use.

9. The speculum (100) of claim 8, wherein the actuation mechanism is a thumb tab (108) and wherein the thumb tab (108) comprises a portion of the lighting module (200).

10. The speculum (100) of claim 8, wherein the lighting element is an LED (204).

11. The speculum (100) of claim 8, wherein the power source is a battery (206).

12. The speculum (100) of claim 8,
wherein the activation mechanism is a pull tab (210) provided between a first and a second battery (206) to prevent discharge of the batteries (206); and
wherein when the pull tab (210) is removed, the first and second batteries (206) become electrically coupled and provide power to the lighting element.

## Patentansprüche

1. Spekulum (100), umfassend:
einen Körperabschnitt, umfassend einen Griff (106), der so konfiguriert ist, dass er von einem Benutzer des Spekulums (100) gegriffen werden kann, sowie ein unteres Schnabelstück (104) und einen Übergangsabschnitt (104a) zwischen dem Griff (106) und dem unteren Schnabelstück (104);
ein oberes Schnabelstück (102), das drehbar mit dem unteren Schnabelstück (104) am Übergangsabschnitt (104a) gekoppelt ist, wodurch sich das obere Schnabelstück (102) relativ zum unteren Schnabelstück (104) bewegen kann, um das Spekulum (100) in eine offene Position zu bewegen;
einen Fensterrahmen (112), der mit dem oberen Schnabelstück (102) gekoppelt ist und ein Sichtfenster definiert;
einen Betätigungsmechanismus, der mit dem oberen Schnabelstück (102) gekoppelt ist, um ein Trennen des Schnabelstücks (102) vom unteren Schnabelstück (104) zu bewirken; und
einen Verriegelungsmechanismus, der so konfiguriert ist, dass er das Spekulum (100) in der offenen Position hält, indem er eine Bewegung des oberen Schnabelstücks (102) in Richtung des unteren Schnabelstücks (104) verhindert, wenn der Verriegelungsmechanismus in Eingriff ist, wobei der Verriegelungsmechanismus Folgendes umfasst:
eine Verriegelungsleiste (124), die in dem Griff (106) angeordnet ist, wobei die Verriegelungsleiste (124) ein Eingriffselement aufweist;
eine Sperrklinke (126) zum Zusammenwirken mit dem Eingriffselement, die beim Zusammenwirken eine Bewegung der Verriegelungsleiste (124) in mindestens einer Richtung relativ zur Sperrklinke (126) verhindert; und
einen Verriegelungsschalter (128), der so konfiguriert ist, dass er bei Betätigung eine Interaktion der Sperrklinke (126) mit dem Eingriffselement bewirkt;
wobei der Übergangsabschnitt (104a) einen Winkel von mehr als 90 Grad zwischen dem Griff (106) und dem unteren Schnabelstück (104) erzeugt.

2. Spekulum (100) nach Anspruch 1, wobei der Winkel im Bereich von 100 Grad bis 180 Grad liegt.

3. Spekulum (100) nach Anspruch 1, wobei das Eingriffselement eine Öffnung (125a) in der Verriegelungsleiste ist, die so konfiguriert ist, dass sie einen Abschnitt der Sperrklinke (126) aufnimmt, der beim Zusammenwirken die Bewegung der Verriegelungsleiste (124) relativ zur Sperrklinke (126) einschränkt.

4. Spekulum (100) nach Anspruch 1, wobei das Eingriffselement ein Zahn (125b) ist, der sich von der Verriegelungsleiste (124) aus erstreckt und so konfiguriert ist, dass er mit einem Abschnitt der Sperrklinke (126) zusammenwirkt, der beim Zusammenwirken eine Bewegung der Verriegelungsleiste (124) in einer Richtung relativ zur Sperrklinke (126) verhindert.

5. Spekulum (100) nach Anspruch 1, wobei sich das obere Schnabelstück (102) beim Lösen des Sperrmechanismus relativ zum unteren Schnabelstück (104) ohne Widerstand und ohne Geräusch bewegt.

6. Spekulum (100) nach Anspruch 1, wobei der Betätigungsmechanismus ein mit dem Fensterrahmen gekoppeltes Daumengriffelement (108) ist.

7. Spekulum (100) nach Anspruch 1, ferner umfassend eine Beleuchtungsquelle.

8. Spekulum (100) nach Anspruch 7, wobei die Beleuchtungsquelle ein Beleuchtungsmodul (200) ist, das Folgendes umfasst:
ein Gehäuse mit einer Frontplatte (208) und einer Rückplatte (202);
ein Beleuchtungselement;
eine Energiequelle; und
einen Aktivierungsmechanismus, um das Beleuchtungselement vor der Verwendung mit Strom zu versorgen.

9. Spekulum (100) nach Anspruch 8, wobei der Betätigungsmechanismus ein Daumengriffelement (108) ist und wobei das Daumengriffelement (108) einen Abschnitt des Beleuchtungsmoduls (200) umfasst.

10. Spekulum (100) nach Anspruch 8, wobei das Beleuchtungselement eine LED (204) ist.

11. Spekulum (100) nach Anspruch 8, wobei die Energiequelle eine Batterie (206) ist.

12. Spekulum (100) nach Anspruch 8, wobei der Aktivierungsmechanismus eine Zuglasche (210) ist, die zwischen einer ersten und einer zweiten Batterie (206) vorgesehen ist, um ein Entladen der Batterien (206) zu verhindern; und
wobei beim Entfernen der Zuglasche (210) die erste und die zweite Batterie (206) elektrisch gekoppelt werden und das Beleuchtungselement mit Strom versorgen.

## Revendications

1. Spéculum (100) comprenant :
une partie de corps comprenant une poignée (106) conçue pour être saisie par un utilisateur du spéculum (100), un bec inférieur (104) et une partie de transition (104a) entre la poignée (106) et le bec inférieur (104) ;
un bec supérieur (102) accouplé de manière rotative au bec inférieur (104) au niveau de la partie de transition (104a), permettant au bec supérieur (102) de se déplacer par rapport au bec inférieur (104) afin de déplacer le spéculum (100) dans une position ouverte ;
un cadre de fenêtre (112) accouplé au bec supérieur (102) définissant une fenêtre de visualisation ;
un mécanisme d'actionnement accouplé au bec supérieur (102) afin de provoquer la séparation du bec supérieur (102) du bec inférieur (104) ; et
un mécanisme de verrouillage conçu pour maintenir le spéculum (100) en position ouverte en empêchant le mouvement du bec supérieur (102) vers le bec inférieur (104) lorsque le mécanisme de verrouillage est entré en prise, le mécanisme de verrouillage comprenant :
une bande de verrouillage (124) contenue à l'intérieur de la poignée (106), la bande de verrouillage (124) possédant un élément de mise en prise ;
un cliquet (126) destiné à interagir avec l'élément de mise en prise qui, lors de l'interaction, empêche le mouvement de la bande de verrouillage (124) dans au moins une direction par rapport au cliquet (126) ; et
un commutateur de verrouillage (128) conçu pour être actionné afin de provoquer l'interaction du cliquet (126) avec l'élément de mise en prise ;
dans lequel la partie de transition (104a) crée un angle supérieur à 90 degrés entre la poignée (106) et le bec inférieur (104).

2. Spéculum (100) selon la revendication 1, dans lequel l'angle est compris entre 100 degrés et 180 degrés.

3. Spéculum (100) selon la revendication 1, dans lequel l'élément de mise en prise est une ouverture (125a) dans la bande de verrouillage conçue pour recevoir une partie du cliquet (126) qui, lors de l'interaction, contraint le mouvement de la bande de verrouillage (124) par rapport au cliquet (126).

4. Spéculum (100) selon la revendication 1, dans lequel l'élément de mise en prise est une dent (125b) s'étendant à partir de la bande de verrouillage (124) conçue pour interagir avec une partie du cliquet (126) qui, lors de l'interaction, empêche le mouvement de la bande de verrouillage (124) dans une direction par rapport au cliquet (126).

5. Spéculum (100) selon la revendication 1, dans lequel lorsque le mécanisme de verrouillage est désengagé, le bec supérieur (102) se déplace par rapport au bec inférieur (104) sans résistance et sans bruit.

6. Spéculum (100) selon la revendication 1, dans lequel le mécanisme d'actionnement est une languette de pouce (108) accouplée au cadre de fenêtre.

7. Spéculum (100) selon la revendication 1, comprenant en outre une source d'éclairage.

8. Spéculum (100) selon la revendication 7, dans lequel la source d'éclairage est un module d'éclairage (200) comprenant :
un boîtier comprenant une plaque avant (208) et une plaque arrière (202) ;
un élément d'éclairage ;
une source d'énergie ; et
un mécanisme d'activation pour alimenter l'élément d'éclairage avant utilisation.

9. Spéculum (100) selon la revendication 8, dans lequel le mécanisme d'actionnement est une languette de pouce (108) et dans lequel la languette de pouce (108) comprend une partie du module d'éclairage (200).

10. Spéculum (100) selon la revendication 8, dans lequel l'élément d'éclairage est une LED (204).

11. Spéculum (100) selon la revendication 8, dans lequel la source d'alimentation est une batterie (206).

12. Spéculum (100) selon la revendication 8,
dans lequel le mécanisme d'activation est une tirette (210) prévue entre une première et une seconde batterie (206) pour empêcher la décharge des batteries (206) ; et
dans lequel lorsque la tirette (210) est retirée, les première et seconde batteries (206) deviennent couplées électriquement et fournissent de l'énergie à l'élément d'éclairage.
